# EUROPÄISCHE PATENTANMELDUNG

(11) **EP 4 512 340 A1**
(43) Veröffentlichungstag der Anmeldung: **26.02.2025**
(21) Anmeldenummer: 23193003.3
(22) Anmeldetag: 23.08.2023
(51) Int. Cl.: A61B 6/03, A61B 6/40, A61B 6/00

(54) **GANTRY FÜR EIN CT-SYSTEM IN C-FORM**

(71) Anmelder: Siemens Healthineers AG, 91301 Forchheim (DE)
(72) Erfinder: Müller, Hans-Jürgen, 91362 Pretzfeld (DE)
(74) Vertreter: Siemens Healthineers Patent Attorneys

(57) **Zusammenfassung**

Die Erfindung betrifft eine Gantry für ein CT-System,
- wobei die Gantry eine Aufnahmekomponente mit einem Strahlungsquellen-Element und einem Detektor-Element aufweist,
- wobei das Strahlungsquellen-Element und das Detektor-Element jeweils in Form eines C-förmigen Kreisringsegments ausgebildet sind, koaxial zu einer Rotationsachse angeordnet sind und in Bezug auf die Rotationsachse jeweils einen Öffnungswinkel des entsprechenden C-förmigen Kreisringsegments zwischen 20° und 180° aufweisen,
- wobei das Strahlungsquellen-Element eine Gruppe von Strahlungsquellen aufweist, wobei eine Strahlungsquelle der Gruppe von Strahlungsquellen an einem ersten Endbereich oder einem zweiten Endbereich des C-förmigen Kreisringsegments des Strahlungsquellen-Elements angeordnet ist,
- wobei das Strahlungsquellen-Element und das Detektor-Element relativ zueinander um die Rotationsachse drehbar gelagert sind.

## Beschreibung

Die Erfindung betrifft eine Gantry für ein CT-System und ein Verfahren zur Aufnahme von CT-Daten mittels einer Gantry. Die Erfindung betrifft ferner eine Steuereinrichtung für ein CT-System und ein CT-System.

Die Computertomographie (kurz: "CT") ist ein etabliertes Bildgebungsverfahren in der Medizin. Bei einer CT-Untersuchung dreht sich die Aufnahmekomponente eines CT-Systems um einen Patienten. Der bei einer Aufnahme überstrichene Winkelbereich beträgt typischerweise 360°, wobei innerhalb dieses Winkelbereichs eine Vielzahl von Projektionsaufnahmen bei unterschiedlichen Winkeln angefertigt wird. Die Anzahl der Projektionsaufnahmen liegt dabei typischerweise in einer Größenordnung von 1000. Aus diesen Projektionsaufnahmen kann dann ein dreidimensionales Bild rekonstruiert werden.

Bei manchen medizinischen Untersuchungen bzw. Anwendungen ist es von Vorteil, wenn die Gantry eines CT-Systems seitlich vom Patienten weggefahren werden kann, oder seitlich über ihn gefahren werden kann, beispielsweise bei einer Interventions-CT oder einer CT an einem OP-Tisch. Dadurch kann die Untersuchung einfacher, schneller und effektiver durchgeführt werden. Dazu muss der geschlossene Kreis der Gantry, bzw. deren Aufnahmekomponente (dem Rotor) mit all ihren Funktionen, insbesondere Lagerung, Antrieb, Datenübertragung, Energieübertragung, Verkleidungen, eines herkömmlichen CT-Systems geöffnet und wieder geschlossen werden können.

Darüber hinaus muss die Konstruktion so gestaltet sein, dass die Strahlungsquelle und auch das Detektormodul (DMS) um den Patienten weiterhin drehbar sind, um vollständige Bilddaten aufzunehmen.

Es ist eine Aufgabe der vorliegenden Erfindung, eine Gantry für ein CT-System zur Verfügung zu stellen, welche einfach seitlich über einen Patienten bewegt werden kann oder in die ein Patient seitlich hineinbewegt werden kann. Insbesondere ist es eine Aufgabe der Erfindung, ein CT-System zu schaffen, dessen Gantry sich öffnen und schließen lässt und insbesondere ein offenes C-Design aufweist. Unabhängig vom grammatikalischen Geschlecht eines bestimmten Begriffes sind Personen mit männlicher, weiblicher oder anderer Geschlechteridentität mit umfasst.

Diese Aufgabe wird durch eine Gantry gemäß Patentanspruch 1, ein Verfahren gemäß Patentanspruch 8, eine Steuereinrichtung gemäß Patentanspruch 14 sowie ein CT-System gemäß Patentanspruch 15 gelöst.

Die Erfindung betrifft eine Gantry für ein CT-System,
- wobei die Gantry eine Aufnahmekomponente mit einem Strahlungsquellen-Element und einem Detektor-Element aufweist,
- wobei das Strahlungsquellen-Element und das Detektor-Element jeweils in Form eines C-förmigen Kreisringsegments ausgebildet sind, koaxial zu einer Rotationsachse angeordnet sind und in Bezug auf die Rotationsachse jeweils einen Öffnungswinkel des entsprechenden C-förmigen Kreisringsegments zwischen 20° und 180° aufweisen,
- wobei das Strahlungsquellen-Element eine Gruppe von Strahlungsquellen aufweist, wobei eine Strahlungsquelle der Gruppe von Strahlungsquellen an einem ersten Endbereich oder einem zweiten Endbereich des C-förmigen Kreisringsegments des Strahlungsquellen-Elements angeordnet ist,
- wobei das Strahlungsquellen-Element und das Detektor-Element relativ zueinander um die Rotationsachse drehbar gelagert sind.

Die Aufnahmekomponente einer herkömmlichen Gantry wird oft als "Rotor" bezeichnet und umfasst die Komponenten, die für die Bildaufnahme zuständig sind, also die Strahlungsquelle und den Detektor.

Insbesondere kann vorgesehen sein, dass sich der Öffnungswinkel des C-förmigen Kreisringsegments des Strahlungsquellen-Elements von dem ersten Endbereich des C-förmigen Kreisringsegments des Strahlungsquellen-Elements bis zu dem zweiten Endbereich des C-förmigen Kreisringsegments des Strahlungsquellen-Elements erstreckt.

Insbesondere kann vorgesehen sein, dass die Strahlungsquellen der Gruppe von Strahlungsquellen gleichmäßig voneinander beabstandet entlang eines Umfangs des C-förmigen Kreisringsegments des Strahlungsquellen-Elements angeordnet sind und/oder in einem gleichmäßigen Winkelabstand voneinander in Bezug auf die Rotationsachse angeordnet sind.

Wenigstens eine der Strahlungsquellen ist im Endbereich der C-Form angeordnet, womit gemeint ist, dass sie bezüglich der Gesamtlänge des Kreissegments innerhalb der ersten und letzten 20%, insbesondere der ersten und letzten 10% der Gesamtlänge angeordnet ist.

Insbesondere kann vorgesehen sein, dass sich ein Zentrum der Aufnahmekomponente, ein Zentrum des C-förmigen Kreisringsegments des Strahlungsquellen-Elements und/oder ein Zentrum des C-förmigen Kreisringsegments des Detektor-Elements auf der Rotationsachse befindet.

Insbesondere kann vorgesehen sein, dass die Rotationsachse zu einem Radius des C-förmigen Kreisringsegments des Strahlungsquellen-Elements und zu einem Radius des C-förmigen Kreisringsegments des Detektor-Elements jeweils orthogonal ist. Die Rotationsachse entspricht im Wesentlichen der Drehachse eines ringförmigen Rotors einer herkömmlichen Gantry insbesondere mit dem Unterschied, dass die Aufnahmekomponente der erfindungsgemäßen Gantry keinen geschlossenen Ring bildet.

Aufgrund der Drehbarkeit des Strahlungsquellen-Elements und der Verwendung mehrerer Strahlungsquellen sind Aufnahmen über einen Winkel von 360° hinweg möglich. Es muss nur darauf geachtet werden, dass sich stets ein Detektor gegenüber dem jeweils emittierten Strahlkegel befindet.

Die Erfindung betrifft ferner ein Verfahren zur Aufnahme von CT-Daten mittels einer erfindungsgemäßen Gantry, wobei das Verfahren die folgenden Schritte umfasst:
- ein Positionieren des Strahlungsquellen-Elements und des Detektor-Elements derart, dass der Öffnungswinkel des C-förmigen Kreisringsegments des Strahlungsquellen-Elements und der Öffnungswinkel des C-förmigen Kreisringsegments des Detektor-Elements sich überlappen und eine seitliche Öffnung der Aufnahmekomponente bilden, wobei die seitliche Öffnung der Aufnahmekomponente in Bezug auf die Rotationsachse radial ausgerichtet ist,
- ein Einführen eines Patienten durch die seitliche Öffnung der Aufnahmekomponente hin zu der Rotationsachse,
- ein Durchführen einer Serie von Projektionsaufnahmen, die zusammen einen Winkelbereich von mindestens 270° um die Rotationsachse abdecken, mit Strahlung aus der Gruppe von Strahlungsquellen des Strahlungsquellen-Elements, wobei das Strahlungsquellen-Element relativ zu dem Patienten gedreht wird,
- wobei innerhalb der Serie von Projektionsaufnahmen eine aktive Strahlungsquelle der Gruppe von Strahlungsquellen mindestens einmal gewechselt wird, indem von einer Strahlungsquelle der Gruppe von Strahlungsquellen auf eine andere Strahlungsquelle der Gruppe von Strahlungsquellen umgeschaltet wird,
- wobei das Detektor-Element durch eine Rotation relativ zu dem Strahlungsquellen-Element so positioniert wird, dass für jede Projektionsaufnahme der Serie von Projektionsaufnahmen die Strahlung der jeweils aktiven Strahlungsquelle von dem Detektor-Element detektiert wird.

Das Positionieren des Strahlungsquellen-Elements und des Detektor-Elements kann insbesondere durch die Rotation des Strahlungsquellen-Elements relativ zu der Halterung und relativ zu dem Detektor-Element um die Rotationsachse und/oder durch die Rotation des Detektor-Elements relativ zu der Halterung und relativ zu dem Strahlungsquellen-Element um die Rotationsachse erfolgen. Beim Positionieren der Aufnahmekomponente werden bevorzugt das Strahlungsquellen-Element und das Detektor-Element in der Aufnahmekomponente so positioniert, dass sich die jeweiligen Öffnungswinkel der Kreissegmente überlappen.

Das Einführen des Patienten durch die seitliche Öffnung der Aufnahmekomponente hin zu der Rotationsachse kann beispielsweise durch ein Schieben der Aufnahmekomponente über den Patienten erfolgen. Aufgrund der seitlichen Öffnung der Aufnahmekomponente kann diese je nach räumlicher Ausrichtung von der Seite (des Patienten), von vorne oder von hinten über den Patienten geschoben werden.

Nun werden das Strahlungsquellen-Element und das Detektor-Element für eine Aufnahme bewegt. Dabei wird während der Aufnahme Strahlung aus den Strahlungsquellen des Strahlungsquellen-Elements für eine Serie von Projektionsaufnahmen in einem Winkelbereich von mindestens 270° (besser über die vollen 360°) durch das Zentrum der Aufnahmekomponente emittiert. Innerhalb der Serie von Projektionsaufnahmen wird dabei die Strahlungsquelle des Strahlungsquellen-Elements mindestens einmal gewechselt. Die gerade emittierende Strahlungsquelle ist die "aktive Strahlungsquelle".

Das Detektor-Element wird für jede Projektionsaufnahme so positioniert, dass die Strahlung der jeweils aktiven Strahlungsquelle von dem Detektor-Element detektiert werden kann. Ist das Strahlungsquellen-Element gleichzeitig das Detektor-Element, so sind die Detektoren bereits korrekt positioniert. In dem besonders bevorzugten Fall, dass sich Strahlungsquellen-Element und Detektor-Element unabhängig voneinander bewegen, sollte das Detektor Element entsprechend der Position der gerade verwendeten Strahlungsquelle passend gedreht werden.

Bei der Bewegung der Elemente sollte darauf geachtet werden, dass möglichst die jeweilige Positionierung so ausgenutzt wird, dass möglichst wenig Drehbewegung notwendig ist. Es sollte ebenfalls darauf geachtet werden, dass die Bewegung von einer einzigen Antriebseinheit, bzw. einer einzigen Halterung, ausgehen kann.

In einem Beispiel, in dem das Strahlungsquellen-Element zwei gegenüberliegende Strahlungsquellen aufweist (also 180° zueinander auf dem Kreisbogen angeordnet), und die Aufnahmekomponente in einer einzigen Halterung gehaltert ist, kann mit einem Detektor-Element gearbeitet werden, welches etwas größer ist als 180° (so dass der Strahlkegel bei voller Auslenkung noch detektiert werden kann). Zuerst wird eine der Strahlungsquellen aktiviert und das Strahlungsquellen-Element über 180° gedreht. Dabei wird das Detektor-Element entsprechend zum Strahlungsquellen-Element bevorzugt so bewegt, so dass die Aufnahmekomponente die ganze Zeit über offen ist. Nach den 180° wird die Strahlenquelle umgeschaltet, so dass nun die andere Strahlenquelle emittiert, das Detektor-Element entsprechend positioniert, so dass die Aufnahmekomponente offenbleibt, und die anderen 180° entsprechend aufgenommen.

Es gibt noch weitere Aufnahmemodi. Besonders bevorzugt ist die Verwendung mehrerer Strahlungsquellen. Damit ist es möglich, dass der Aufnahmebereich nicht durch Drehung des Strahlungsquellen-Elements abgefahren werden muss, sondern (zumindest auch) durch das Wechseln von Strahlungsquellen. Dies wird weiter unten ausführlicher beschrieben.

Die Erfindung betrifft ferner eine Steuereinrichtung für ein CT-System, wobei die Steuereinrichtung zum Steuern einer erfindungsgemäßen Gantry gemäß einem erfindungsgemäßen Verfahren eingerichtet ist. Die Erfindung betrifft ferner ein CT-System, umfassend eine erfindungsgemäße Gantry und eine erfindungsgemäße Steuereinrichtung.

Hiermit wird ferner eine Gantry für ein CT-System offenbart,
- wobei die Gantry eine Aufnahmekomponente mit einem Strahlungsquellen-Element und einem Detektor-Element aufweist,
- wobei das Strahlungsquellen-Element und das Detektor-Element jeweils in Form eines C-förmigen Kreisringsegments ausgebildet sind, koaxial zu einer Rotationsachse angeordnet sind und in Bezug auf die Rotationsachse jeweils einen Öffnungswinkel des entsprechenden C-förmigen Kreisringsegments zwischen 20° und 180° aufweisen,
- wobei das Strahlungsquellen-Element eine Gruppe von Strahlungsquellen aufweist, wobei eine Strahlungsquelle der Gruppe von Strahlungsquellen an einem ersten Endbereich oder einem zweiten Endbereich des C-förmigen Kreisringsegments des Strahlungsquellen-Elements angeordnet ist,
- wobei das Strahlungsquellen-Element und das Detektor-Element relativ zueinander fest angeordnet sind und zusammen relativ zu einer Halterung der Gantry um die Rotationsachse drehbar gelagert sind. Diese Gantry kann nach einem der hier beschriebenen Aspekte, der nicht von einer Drehung des Strahlungsquellen-Elements und das Detektor-Elements relativ zueinander abhängt, ausgebildet sein.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bei einer bevorzugten Gantry sind Detektor-Element und Strahlungsquellen-Element koaxial zu einer gemeinsamen Rotationsachse orthogonal zu den Radien der Kreisringsegmente so angeordnet, dass sie um die Rotationsachse unabhängig voneinander drehbar sind. Dies stellt eine besonders bevorzugte Ausführungsform dar, bei der insbesondere mehrere Strahlungsquellen verwendet werden können und eine Detektorfläche stets optimal positioniert werden kann.

Eine Ausführungsform sieht vor, dass der Öffnungswinkel jeweils mindestens 30° ist, bevorzugt mindestens 60°, bevorzugt jedoch maximal 140° ist. Damit kann ein Patient bequem seitlich eingebracht werden bzw. die Aufnahmekomponente einfach seitlich, von vorne oder von hinten über einen Patienten geschoben werden kann. Der Radius entspricht dabei bevorzugt herkömmlichen (geschlossenen) Geräten, so dass bei diesem Öffnungswinkel die Öffnung groß genug ist. Der Öffnungswinkel sollte aber nicht größer als 140° sein, bevorzugt nicht größer als 120°, damit die Aufnahmen mit möglichst wenig Aufwand angefertigt werden können.

Eine Ausführungsform sieht vor, dass der Öffnungswinkel des C-förmigen Kreisringsegments des Strahlungsquellen-Elements größer ist als der Öffnungswinkel des C-förmigen Kreisringsegments des Detektor-Elements. Die Strahlungsquellen emittieren in der Regel Strahlkegel, sodass der Detektor eine größere Fläche überdecken sollte als die Abmessung der Strahlungsquellen.

Eine Ausführungsform sieht vor, dass die Gruppe von Strahlungsquellen mindestens zehn Strahlungsquellen umfasst, insbesondere mindestens 36 Strahlungsquellen umfasst, insbesondere 100 oder mehr Strahlungsquellen umfasst. Je mehr Strahlungsquellen vorhanden sind, desto weniger Bewegung ist nötig, um genügend Projektionsaufnahmen anzufertigen. Bevorzugt sind die Strahlungsquellen der Gruppe von Strahlungsquellen einzeln ansteuerbar.

Die Strahlungsquellen sind bevorzugt in regelmäßigen Abständen angeordnet. Dies hat den Vorteil, dass ohne eine Drehung des Strahlungsquellen-Elements Aufnahmen aus einer Vielzahl von Aufnahmewinkeln erfolgen können.

Eine Ausführungsform sieht vor, dass die Strahlungsquellen der Gruppe von Strahlungsquellen Kohlenstoffnanoröhren-Feldemitter (Nano Tube Field Emitter) sind. Diese sind klein, preisgünstig, und emittieren eine optimale Strahlung.

Das Detektor-Element kann beispielsweise mehrere Detektoren und/oder einen über die Länge des Kreisrings ausgedehnten Detektor aufweisen. Im Grunde muss ein Detektor lediglich so groß sein, dass er den Strahlenkegel abdecken kann bzw. den gewünschten Aufnahmebereich abdeckt. In diesem Falle sollte der Detektor jedoch innerhalb des Detektor-Elements verschiebbar sein, dass es über die Länge des Kreisbogens des Detektor-Elements an die jeweils benötigte Aufnahmeposition bewegt werden kann. Das Detektor-Element kann insbesondere für spektral aufgelöste und/oder photonenzählende Detektion von Röntgenstrahlung eingerichtet sein.

Eine Ausführungsform sieht vor, dass die Gantry eine Halterung aufweist, wobei die Aufnahmekomponente an der Halterung gehaltert ist, wobei das Strahlungsquellen-Element und das Detektor-Element unabhängig voneinander jeweils relativ zu der Halterung um die Rotationsachse drehbar gelagert sind. Bevorzugt ist diese Halterung nur in einem Halterungswinkelbereich in Bezug auf die Rotationsachse ausgeformt und haltert die Aufnahmekomponente nur in diesem Halterungswinkelbereich.

Eine Ausführungsform sieht vor, dass die Gantry eine Antriebseinheit aufweist, wobei die Antriebseinheit dazu eingerichtet ist, eine Rotation des Strahlungsquellen-Elements relativ zu der Halterung und relativ zu dem Detektor-Element um die Rotationsachse anzutreiben und/oder eine Rotation des Detektor-Elements relativ zu der Halterung und relativ zu dem Strahlungsquellen-Element um die Rotationsachse anzutreiben, insbesondere durch eine Kraftausübung auf eine Antriebsleiste, eine Mantelfläche und/oder eine Stirnfläche des entsprechenden C-förmigen Kreisringsegments anzutreiben, beispielweise mittels eines Zahnrads, das auf einer gezahnten Leiste an dem jeweiligen Element läuft.

Mit einer solchen Halterung können die Elemente der Aufnahmekomponente einfach unabhängig zueinander bewegt werden. Eine Ausführungsform sieht vor, dass das Strahlungsquellen-Element und das Detektor-Element so relativ zueinander gedreht werden, dass bei jeder Projektionsaufnahme der Serie von Projektionsaufnahmen ein Strahlkegel der aktiven Strahlungsquelle nach einer Wechselwirkung mit dem Patienten auf einen für diese Projektionsaufnahme verwendeten Bereich eines Detektors des Detektor-Elements trifft.

Eine Ausführungsform sieht vor, dass zu Beginn der Serie von Projektionsaufnahmen das Strahlungsquellen-Element so positioniert wird, dass es an dem ersten Endbereich des C-förmigen Kreisringsegments des Strahlungsquellen-Elements von der Halterung gehalten wird,
- wobei während einer ersten Teilserie der Serie von Projektionsaufnahmen das Strahlungsquellen-Element relativ zu der Halterung um die Rotationsachse so weit gedreht wird, bis es an dem zweiten Endbereich des C-förmigen Kreisringsegments des Strahlungsquellen-Elements von der Halterung gehalten wird, wobei spätestens dann die aktive Strahlungsquelle der Gruppe von Strahlungsquellen gewechselt wird,
- wobei während einer zweiten Teilserie der Serie von Projektionsaufnahmen das Strahlungsquellen-Element relativ zu der Halterung um die Rotationsachse so weit zurückgedreht wird, bis es wieder an dem ersten Endbereich des C-förmigen Kreisringsegments des Strahlungsquellen-Elements von der Halterung gehalten wird.

Insbesondere kann vorgesehen sein, dass die zweite Teilserie der Serie von Projektionsaufnahmen auf die erste Teilserie der Serie von Projektionsaufnahmen folgt.

Eine Ausführungsform sieht vor, dass innerhalb der Serie von Projektionsaufnahmen die aktive Strahlungsquelle der Gruppe von Strahlungsquellen mehrfach gewechselt wird, während sich das Strahlungsquellen-Element relativ zu der Halterung um die Rotationsachse dreht. Dadurch kann die Rotationsgeschwindigkeit des Strahlungsquellen-Elements vergleichsweise gering sein.

Eine Ausführungsform sieht vor, dass innerhalb der Serie von Projektionsaufnahmen das Detektor-Element relativ zu der Halterung um die Rotationsachse um einen Detektor-Drehwinkel, der mindestens so groß wie der Öffnungswinkel des C-förmigen Kreisringsegments des Detektor-Elements ist, gedreht wird, insbesondere während sich das Strahlungsquellen-Element relativ zu der Halterung um die Rotationsachse dreht.

Die Serie von Projektionsaufnahmen kann insbesondere Projektionsaufnahmen für einen Projektionswinkelbereich in Bezug auf die Rotationsachse umfassen, in dem sich die seitliche Öffnung der Aufnahmekomponente während des Einführens des Patienten befindet. Beispielsweise kann durch die Rotation des Detektor-Elements relativ zu der Halterung um die Rotationsachse ein Detektor des Detektor-Elements in diesen Projektionswinkelbereich hineingefahren werden, um diesen Projektionswinkelbereich zu überdecken.

Bevorzugt wird dabei das Detektor-Element währenddessen so verfahren, dass dessen Öffnungswinkel zu Beginn der Aufnahme durch die Bewegung von einem Detektor des Detektor-Elements später während der Aufnahme überdeckt wird. Im Grunde ist der Bereich, in dem sich der Öffnungswinkel des Detektor-Elements zu Beginn der Aufnahme befand, derjenige, in dem zunächst keine Aufnahmen gemacht werden können. Wird dieser später überdeckt, dann können dort Aufnahmen gemacht werden. Insbesondere kann vorgesehen sein, dass innerhalb der Serie von Projektionsaufnahmen das Strahlungsquellen-Element relativ zu der Halterung um die Rotationsachse um einen Strahlungsquellen-Drehwinkel, der mindestens so groß wie der Öffnungswinkel des C-förmigen Kreisringsegments des Strahlungsquellen-Elements ist, gedreht wird.

Eine Ausführungsform sieht vor, dass die Halterung dazu eingerichtet ist, die Aufnahmekomponente zu verschwenken, wobei die Aufnahmekomponente vor einer Untersuchung eines liegenden Patienten mittels der Halterung verschwenkt wird, bis die Rotationsachse eine horizontale Ausrichtung erreicht, und/oder wobei die Aufnahmekomponente vor einer Untersuchung eines stehenden Patienten mittels der Halterung verschwenkt wird, bis die Rotationsachse eine vertikale Ausrichtung erreicht.

Bevorzugt ist die Halterung zusätzlich dazu ausgelegt, die Rotationsachse der Aufnahmekomponente zu verschwenken, also die Aufnahmekomponente zu einer Achse quer (insbesondere orthogonal) zur Rotationsachse zu drehen. Bevorzugt soll die Verschwenkung so erfolgen, dass die Rotationsachse zwischen einer horizontalen Ausrichtung und einer vertikalen Ausrichtung wechseln kann.

Es ist bevorzugt, dass sich die Halterung an einem Wagen oder an einem Roboterarm befindet. dadurch kann die Gantry mobil gestaltet werden. Durch eine mögliche Verschwenkung der Aufnahmekomponente können sowohl liegende als auch stehende Patienten untersucht werden. Die Gantry kann aber auch fest an einer Wand, auf einem Boden oder an einer Decke montiert sein. Ein Patient kann trotzdem sehr einfach von der Seite in den Untersuchungsbereich der Gantry bewegt werden, z.B. auf einer Patientenliege ruhend oder in einem entsprechend ausgestalteten Bett.

Dabei ist bevorzugt, die Rotationsachse der Aufnahmekomponente zu so verschwenken, dass die Aufnahmekomponente vor der Untersuchung eines liegenden Patienten so positioniert wird, dass ihre Rotationsachse eine horizontale Ausrichtung hat, und die Aufnahmekomponente vor der Untersuchung eines stehenden Patienten so positioniert wird, dass ihre Rotationsachse eine vertikale Ausrichtung hat.

Eine Ausführungsform sieht vor, dass das Strahlungsquellen-Element und das Detektor-Element zueinander entlang der Rotationsachse axial versetzt angeordnet sind und/oder dass das Strahlungsquellen-Element und das Detektor-Element derart unterschiedlich große Radien der entsprechenden C-förmigen Kreisringsegmente aufweisen, dass sie zueinander in Bezug auf die Rotationsachse radial versetzt angeordnet sind.

Bevorzugt sind das Strahlungsquellen-Element und das Detektor-Element zueinander entlang der Rotationsachse verschoben. Dies hat den Vorteil, dass sich ein Detektor des Detektor-Elements im selben Winkelbereich einer Strahlungsquelle des Strahlungsquellen-Elements aufhalten kann, ohne dass die Strahlung der Strahlungsquelle den Detektor trifft (sondern den gegenüberliegenden Detektor). Die Strahlungsquellen sind dabei bevorzugt so positioniert, dass sie einen Strahl etwas schräg (auf den verschobenen Detektor gegenüber) emittieren.

Bevorzugt haben das Strahlungsquellen-Element und das Detektor-Element unterschiedliche Radien, lassen sich also ineinander zueinander drehen.

Eine bevorzugte Gantry für ein CT-System umfasst eine Aufnahmekomponente, die mit zwei C-förmigen Strahlungsquellen-Elementen ausgestattet ist, die zusätzlich Detektoren umfassen (also auch gleichzeitig Detektor-Elemente sind). Diese beiden Strahlungsquellen-Elemente ergeben zusammen bevorzugt 360°, was jedoch nicht zwingend notwendig ist. Jedes der Strahlungsquellen-Elemente umfasst eine Gruppe von Strahlungsquellen, die unabhängig voneinander aktiviert werden können. Ein Detektorbereich erstreckt sich bevorzugt über die gesamte Länge des Strahlungsquellen-Elements. Bevorzugt sind beide Strahlungsquellen-Elemente koaxial um eine gemeinsame Rotationsachse orthogonal zu ihren Radien drehbar angeordnet. Bei einer Verwendung einer Vielzahl von Strahlungsquellen kann diese Ausführungsform auf eine Rotation vollständig verzichten, zumindest, wenn sich der Detektorbereich beider Strahlungsquellen-Elemente insgesamt über 360° erstreckt. In dem bevorzugten Fall, in dem jedes Element sich über 180° erstreckt (also einen Öffnungswinkel von 180° hat), oder eines der Strahlungsquellen-Elemente als Öffnung dient, müssen die beiden Strahlungsquellen-Elemente nur so verdreht werden, dass sich eine seitliche Öffnung in der Gantry ergibt, dann werden sie geschlossen, so dass sich ein kompletter Ring ergibt. Durch Aktivieren unterschiedlicher Strahlungsquellen kann dann (ggf. auch ohne Rotation) eine vollständige CT-Aufnahme angefertigt werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich.
Figur 1 zeigt eine schematische Darstellung eines CT-Geräts gemäß dem Stand der Technik.
Figur 2 zeigt eine Ansicht einer Gantry mit einer Aufnahmekomponente.
Figur 3 zeigt eine weitere Ansicht der Gantry.
Figur 4 zeigt verschiedene Zustände der Gantry während eines Verfahrens zur Aufnahme von CT-Daten mittels der Gantry.
Figur 5 zeigt die Verschwenkung einer Gantry.
Figur 6 zeigt eine Gantry auf einem Wagen.
Figur 7 zeigt eine Gantry an einem Roboterarm.
Figur 8 zeigt eine Gantry an einer Wand.
Figur 9 zeigt eine Gantry auf einem Boden.

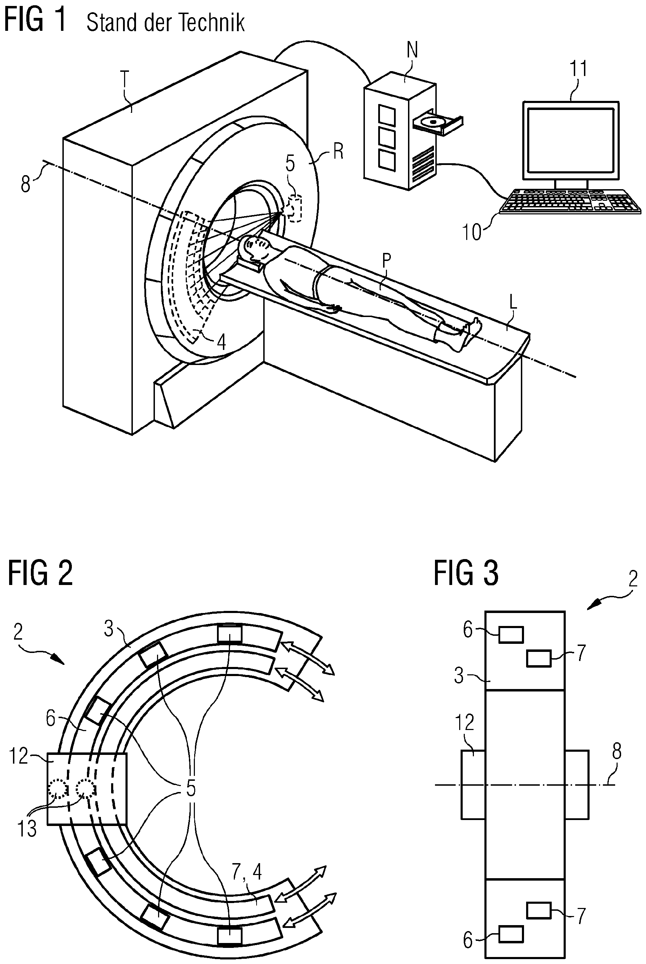

Figur 1 zeigt ein Computertomographie-Gerät (CT-Gerät) mit einer Strahlungsquelle 5 zur Emission von Röntgenstrahlung und einem Detektor 4 zur Detektion der Röntgenstrahlung. Die Strahlungsquelle 5 ist dazu ausgebildet, den Detektor 4 mit Strahlung zu belichten. Das gezeigte CT-Gerät umfasst die Tragstruktur T und den geschlossenen, O-förmigen Rotor R. Der Rotor R umfasst als Strahlungsquelle 5 eine Röntgenquelle und den Detektor 4, welcher ausgebildet ist, Röntgenstrahlung zu detektieren.

Der Rotor R ist relativ zu der Tragstruktur T um die Rotationsachse 8 drehbar gelagert. Der Patient P ist auf der Patientenliege L entlang der Rotationsachse 8 relativ zu der Tragstruktur T bewegbar gelagert. Zur Steuerung des CT-Systems 1 und/oder zur Erzeugung eines Bilddatensatzes basierend auf vom Strahlungsdetektor 4 detektierten Signalen ist die Recheneinheit N vorgesehen.

Es wird üblicherweise aus einer Vielzahl an Winkelrichtungen ein (Roh-) Röntgen-bilddatensatz des Patienten P mittels des Strahlungsdetektors 4 aufgenommen. Anschließend kann basierend auf dem (Roh-) Röntgenbilddatensatz mittels eines mathematischen Verfahrens, beispielsweise umfassend eine ge-filterte Rückprojektion oder ein iteratives Rekonstruktions-verfahren, ein (finaler) Bilddatensatz rekonstruiert werden.

Die Recheneinheit N dient hier als Steuereinrichtung zur Steuerung des CT-Geräts. Mit der Recheneinheit N ist eine Eingabeeinrichtung 10 und eine Ausgabeeinrichtung 11 verbunden. Die Eingabeeinrichtung 10 und die Ausgabeeinrichtung 11 können beispielsweise eine Interaktion durch einen Anwender oder die Darstellung eines erzeugten Bilddatensatzes ermöglichen.

Figur 2 zeigt eine Ansicht einer Gantry 2 mit der Aufnahmekomponente 3. Die Aufnahmekomponente 3 umfasst ein Strahlungsquellen-Element 6 mit einer Vielzahl von regelmäßig angeordneten Strahlungsquellen 5, z.B. Nano Tube Field Emitter, und ein Detektor-Element 7 mit einem über dessen Kreisbogen ausgedehnten Detektor 4. Beide Elemente haben die Form eines C-förmigen Kreisringsegments mit einem recht großen Öffnungswinkel, damit einfach von der Seite über einen Patienten gefahren werden kann.

Detektor-Element 7 und Strahlungsquellen-Element 6 sind koaxial zu einer gemeinsamen Rotationsachse 8 orthogonal zu den Radien der Kreisringsegmente so angeordnet, dass sie um die Rotationsachse 8 unabhängig voneinander drehbar sind (Pfeile). Für diese Bewegung sind die Aufnahmekomponente 3 an einer Halterung 12 gehaltert ist, die eine Antriebseinheit 13 umfasst, welche dazu ausgelegt ist diese Elemente 6, 7 unabhängig voneinander um die Rotationsachse 8 zu verdrehen.

Figur 3 zeigt eine weitere Ansicht der Gantry. Hier ist zu erkennen, dass Strahlungsquellen-Element 6 und Detektor-Element 7 zueinander entlang der Rotationsachse 8 verschoben angeordnet sind und unterschiedliche Radien haben. Die verschobene Anordnung dient dazu, dass kein Detektor 4 direkt vor einer Strahlungsquelle 5 liegen kann und diese verdeckt.

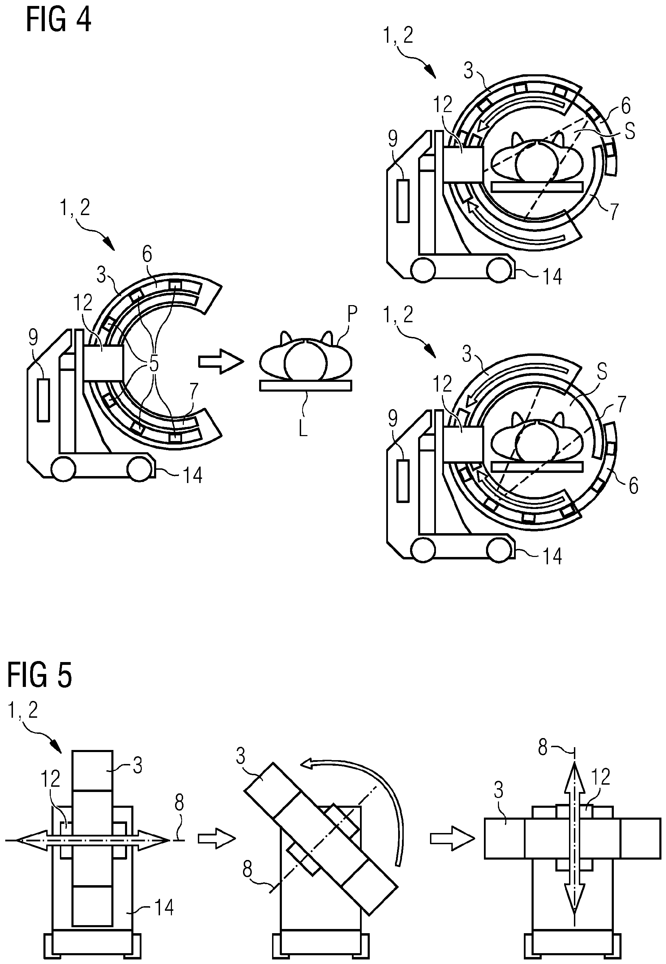

Figur 4 zeigt verschiedene Zustände der Gantry 2 während eines Verfahrens zur Aufnahme von CT-Daten mittels der Gantry 2 des CT-Systems 1. Das CT-System 1 weist die Steuereinrichtung 9 auf.

Links wird die Aufnahmekomponente 3 in einer ÖffnungsPosition über einen Patienten P geschoben. Dann wird, wie rechts angedeutet das Strahlungsquellen-Element 6 und das Detektor-Element 5 für eine Aufnahme so über den Patienten bewegt, dass während der Aufnahme Strahlung aus den Strahlungsquellen 5 des Strahlungsquellen-Elements 6 für eine Serie von Projektionsaufnahmen in einem Winkelbereich von 360° durch das Zentrum der Aufnahmekomponente 3 verläuft.

Die in der Halterung 12 gehalterten Elemente 6, 7 werden durch die Antriebseinheit 13 der Halterung 12 unabhängig voneinander um die Rotationsachse 8 verdreht. Während der Aufnahme werden dabei das Strahlungsquellen-Element 6 und das Detektor-Element 7 so positioniert, dass sich bei jeder Aufnahme ein Strahlkegel S einer für die Aufnahme verwendeten Strahlungsquelle 5 auf einen Bereich eines Detektors 4 des Detektor-Elements 7 trifft.

Innerhalb der Serie von Projektionsaufnahmen wird die aktive Strahlungsquelle 5 mindestens einmal gewechselt (vgl. Darstellungen rechts oben und unten). Das Detektor-Element 7 wird dabei für jede Projektionsaufnahme so positioniert, dass die Strahlung der jeweils aktiven Strahlungsquelle 5 von dem Detektor-Element 7 detektiert werden kann.

Die Gantry 2 ist über ihre Halterung 12 auf einem Wagen 14 montiert, so dass sie mobil ist.

Figur 5 zeigt die Verschwenkung einer Gantry 2, bei der die Halterung 12 zusätzlich dazu ausgelegt ist, die Rotationsachse 8 der Aufnahmekomponente 3 zu verschwenken, bevorzugt so, dass die Rotationsachse 8 zwischen einer horizontalen Ausrichtung (links) und einer vertikalen Ausrichtung (rechts) wechseln kann.

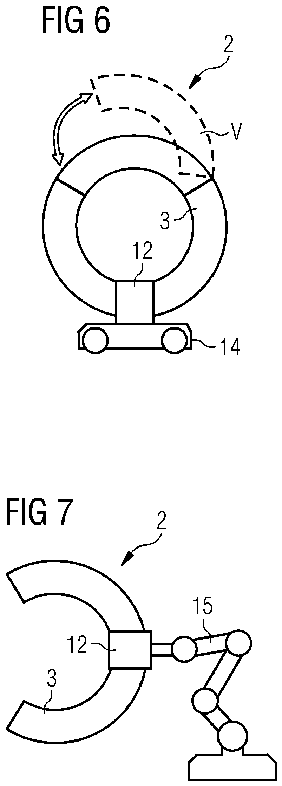

Figur 6 zeigt eine mobile Gantry 2 auf einem Wagen 14. Diese Gantry ist verschlossen, jedoch befindet sich in der Öffnungs-Position kein Element 6, 7 der Aufnahmekomponente 3 hinter der schwenkbaren Klappe. Diese ist lediglich eine Verkleidung V.

Figur 7 zeigt die Gantry 2 an einem Roboterarm 15.

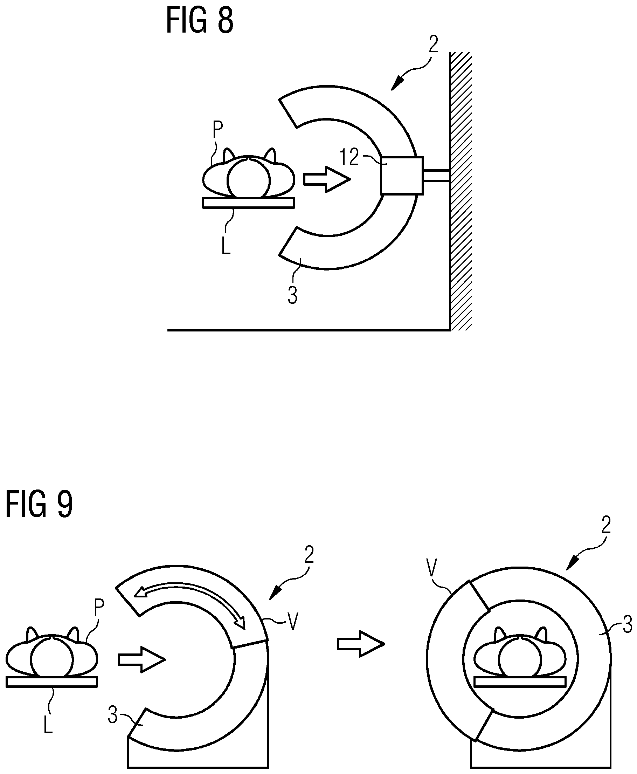

Figur 8 zeigt die Gantry 2, die an einer Wand befestigt ist. Sie könnte auch am Boden oder an der Decke befestigt sein. Ein Patient P kann einfach samt Patientenliege L von der Seite in die Gantry 2 geschoben werden. Dies hat den Vorteil, dass ein Patient P mit einem geeigneten Bett einfach ohne umzubetten untersucht werden kann.

Figur 9 zeigt die Gantry 2, die auf dem Boden befestigt ist. Sie könnte auch an der Wand oder an der Decke befestigt sein.

Sie weist bei einer Untersuchung (rechts) einen geschlossenen Ring auf. Der Abschluss der im Grunde offenen Aufnahmekomponente 3 wird hier durch eine Verkleidung V erreicht, die auf der Wandung der Aufnahmekomponente 3 so verschoben werden kann, dass der Ring für die Einbringung eines Patienten P einfach geöffnet (links) und danach wieder verschlossen werden kann (rechts).

Es wird abschließend noch einmal darauf hingewiesen, dass es sich bei den vorhergehend detailliert beschriebenen Figuren lediglich um Ausführungsbeispiele handelt, welche vom Fach-mann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vor-handen sein können. Ebenso schließen die Begriffe "Einheit" und "Gerät" nicht aus, dass die betreffenden Komponenten aus mehreren zusammenwirkenden Teil-Komponenten bestehen können, die gegebenenfalls auch räumlich verteilt sein können. Der Ausdruck "eine Anzahl" ist als "mindestens eins" zu verstehen.

## Patentansprüche

1. Gantry (2) für ein CT-System (1),
- wobei die Gantry (2) eine Aufnahmekomponente (3) mit einem Strahlungsquellen-Element (6) und einem Detektor-Element (7) aufweist,
- wobei das Strahlungsquellen-Element (6) und das Detektor-Element (7) jeweils in Form eines C-förmigen Kreisringsegments ausgebildet sind, koaxial zu einer Rotationsachse (8) angeordnet sind und in Bezug auf die Rotationsachse (8) jeweils einen Öffnungswinkel des entsprechenden C-förmigen Kreisringsegments zwischen 20° und 180° aufweisen,
- wobei das Strahlungsquellen-Element (6) eine Gruppe von Strahlungsquellen (5) aufweist, wobei eine Strahlungsquelle der Gruppe von Strahlungsquellen (5) an einem ersten Endbereich oder einem zweiten Endbereich des C-förmigen Kreisringsegments des Strahlungsquellen-Elements (6) angeordnet ist,
- wobei das Strahlungsquellen-Element (6) und das Detektor-Element (7) relativ zueinander um die Rotationsachse (8) drehbar gelagert sind.

2. Gantry nach Anspruch 1,
- wobei die Gantry (2) eine Halterung (12) aufweist, wobei die Aufnahmekomponente (3) an der Halterung (12) gehaltert ist,
- wobei das Strahlungsquellen-Element (6) und das Detektor-Element (7) unabhängig voneinander jeweils relativ zu der Halterung (12) um die Rotationsachse (8) drehbar gelagert sind.

3. Gantry nach Anspruch 2,
- wobei die Gantry (2) eine Antriebseinheit (13) aufweist, wobei die Antriebseinheit (13) dazu eingerichtet ist, eine Rotation des Strahlungsquellen-Elements (6) relativ zu der Halterung (12) und relativ zu dem Detektor-Element (7) um die Rotationsachse (8) anzutreiben und/oder eine Rotation des Detektor-Elements (7) relativ zu der Halterung (12) und relativ zu dem Strahlungsquellen-Element (6) um die Rotationsachse (8) anzutreiben.

4. Gantry (2) nach einem der Ansprüche 1 bis 3,
- wobei die Gruppe von Strahlungsquellen (5) mindestens zehn Strahlungsquellen umfasst.

5. Gantry (2) nach einem der Ansprüche 1 bis 4,
- wobei die Strahlungsquellen der Gruppe von Strahlungsquellen (5) Kohlenstoffnanoröhren-Feldemitter sind.

6. Gantry nach einem der Ansprüche 1 bis 5,
- wobei der Öffnungswinkel jeweils mindestens 30° ist, bevorzugt mindestens 60°, bevorzugt jedoch maximal 140° ist, und/oder
- wobei der Öffnungswinkel des C-förmigen Kreisringsegments des Strahlungsquellen-Elements (6) größer ist als der Öffnungswinkel des C-förmigen Kreisringsegments des Detektor-Elements (7).

7. Gantry nach einem der Ansprüche 1 bis 6,
- wobei das Strahlungsquellen-Element (6) und das Detektor-Element (7) zueinander entlang der Rotationsachse (8) axial versetzt angeordnet sind und/oder
- wobei das Strahlungsquellen-Element (6) und das Detektor-Element (7) derart unterschiedlich große Radien der entsprechenden C-förmigen Kreisringsegmente aufweisen, dass sie zueinander in Bezug auf die Rotationsachse (8) radial versetzt angeordnet sind.

8. Verfahren zur Aufnahme von CT-Daten mittels einer Gantry (2) nach einem der Ansprüche 1 bis 7, wobei das Verfahren die folgenden Schritte umfasst:
- ein Positionieren des Strahlungsquellen-Elements (6) und des Detektor-Elements (7) derart, dass der Öffnungswinkel des C-förmigen Kreisringsegments des Strahlungsquellen-Elements (6) und der Öffnungswinkel des C-förmigen Kreisringsegments des Detektor-Elements (7) sich überlappen und eine seitliche Öffnung der Aufnahmekomponente (3) bilden, wobei die seitliche Öffnung der Aufnahmekomponente (3) in Bezug auf die Rotationsachse (8) radial ausgerichtet ist,
- ein Einführen eines Patienten (P) durch die seitliche Öffnung der Aufnahmekomponente (3) hin zu der Rotationsachse (8),
- ein Durchführen einer Serie von Projektionsaufnahmen, die zusammen einen Winkelbereich von mindestens 270° um die Rotationsachse (8) abdecken, mit Strahlung aus der Gruppe von Strahlungsquellen (5) des Strahlungsquellen-Elements (6), wobei das Strahlungsquellen-Element (6) relativ zu dem Patienten (P) gedreht wird,
- wobei innerhalb der Serie von Projektionsaufnahmen eine aktive Strahlungsquelle der Gruppe von Strahlungsquellen (5) mindestens einmal gewechselt wird, indem von einer Strahlungsquelle der Gruppe von Strahlungsquellen (5) auf eine andere Strahlungsquelle der Gruppe von Strahlungsquellen (5) umgeschaltet wird,
- wobei das Detektor-Element (7) durch eine Rotation relativ zu dem Strahlungsquellen-Element (6) so positioniert wird, dass für jede Projektionsaufnahme der Serie von Projektionsaufnahmen die Strahlung der jeweils aktiven Strahlungsquelle der Gruppe von Strahlungsquellen (5) von dem Detektor-Element (7) detektiert wird.

9. Verfahren nach Anspruch 8,
- wobei das Strahlungsquellen-Element (6) und das Detektor-Element (7) so relativ zueinander gedreht werden, dass bei jeder Projektionsaufnahme der Serie von Projektionsaufnahmen ein Strahlkegel (S) der aktiven Strahlungsquelle der Gruppe von Strahlungsquellen (5) nach einer Wechselwirkung mit dem Patienten (P) auf einen für diese Projektionsaufnahme verwendeten Bereich eines Detektors (4) des Detektor-Elements (7) trifft.

10. Verfahren nach Anspruch 8 oder 9,
- wobei zu Beginn der Serie von Projektionsaufnahmen das Strahlungsquellen-Element (6) so positioniert wird, dass es an dem ersten Endbereich des C-förmigen Kreisringsegments des Strahlungsquellen-Elements (6) von der Halterung (12) gehalten wird,
- wobei während einer ersten Teilserie der Serie von Projektionsaufnahmen das Strahlungsquellen-Element (6) relativ zu der Halterung (12) um die Rotationsachse (8) so weit gedreht wird, bis es an dem zweiten Endbereich des C-förmigen Kreisringsegments des Strahlungsquellen-Elements (6) von der Halterung (12) gehalten wird, wobei spätestens dann die aktive Strahlungsquelle der Gruppe von Strahlungsquellen (5) gewechselt wird,
- wobei während einer zweiten Teilserie der Serie von Projektionsaufnahmen das Strahlungsquellen-Element (6) relativ zu der Halterung (12) um die Rotationsachse (8) so weit zurückgedreht wird, bis es wieder an dem ersten Endbereich des C-förmigen Kreisringsegments des Strahlungsquellen-Elements (6) von der Halterung (12) gehalten wird.

11. Verfahren nach einem der Ansprüche 8 bis 10,
- wobei innerhalb der Serie von Projektionsaufnahmen die aktive Strahlungsquelle der Gruppe von Strahlungsquellen (5) mehrfach gewechselt wird, während sich das Strahlungsquellen-Element (6) relativ zu der Halterung (12) um die Rotationsachse (8) dreht.

12. Verfahren nach einem der Ansprüche 8 bis 11,
- wobei innerhalb der Serie von Projektionsaufnahmen das Detektor-Element (7) relativ zu der Halterung (12) um die Rotationsachse (8) um einen Detektor-Drehwinkel, der mindestens so groß wie der Öffnungswinkel des C-förmigen Kreisringsegments des Detektor-Elements (7) ist, gedreht wird.

13. Verfahren nach einem der Ansprüche 8 bis 12,
- wobei die Halterung (12) dazu eingerichtet ist, die Aufnahmekomponente (3) zu verschwenken,
- wobei die Aufnahmekomponente (3) vor einer Untersuchung eines liegenden Patienten (P) mittels der Halterung (12) verschwenkt wird, bis die Rotationsachse (8) eine horizontale Ausrichtung erreicht, und/oder wobei die Aufnahmekomponente (3) vor einer Untersuchung eines stehenden Patienten (P) mittels der Halterung (12) verschwenkt wird, bis die Rotationsachse (8) eine vertikale Ausrichtung erreicht.

14. Steuereinrichtung (9) für ein CT-System (1), eingerichtet zum Steuern einer Gantry (2) nach einem der Ansprüche 1 bis 7 gemäß einem Verfahren nach einem der Ansprüche 8 bis 13.

15. CT-System (1), umfassend eine Gantry (2) nach einem der Ansprüche 1 bis 7 und eine Steuereinrichtung (9) nach Anspruch 14.
